# EUROPEAN PATENT SPECIFICATION

(11) **EP 4 408 488 B1**
(45) Date of publication and mention of the grant of the patent: **27.08.2025**
(21) Application number: 22786790.0
(22) Date of filing: 16.09.2022
(51) Int. Cl.: A61L 2/22, B08B 9/032, B65B 55/00

(54) **APPARATUS FOR DISINFECTION OF CLOSED SPACES**
VORRICHTUNG ZUR DESINFEKTION VON GESCHLOSSENEN RÄUMEN
APPAREIL DE DÉSINFECTION D'ESPACES FERMÉS

(30) Priority: 29.09.2021 IS 9155
(43) Date of publication of application: 07.08.2024
(73) Proprietor: Alvar Mist ehf, IS203 Kopavogur (IS)
(72) Inventor: OLAFSSON, Ragnar, Reykjavik (IS); PECIULAITIS, Lukas, Hafnarfjordur (IS)
(74) Representative: KIPA AB
(86) International application number: PCT/EP2022/075811
(87) International publication number: WO 2023/052153

(56) References cited:
- DE-A1- 102009 026 039
- JP-A- H0 958 632
- US-A- 4 537 749
- US-A1- 2017 173 199

## Description

### FIELD

The invention relates to an apparatus that contains one or more atomizing or spray units for sanitizing or disinfecting closed spaces such as food transport systems or food containers. The invention also relates to systems comprising a sanitizing apparatus and methods of sanitizing or disinfecting using such apparatus.

### INTRODUCTION

Many different spaces are used for storing, processing or transporting of various sensitive biological tissues or living organisms, such as living fish or components of fish, including offal. Often such sensitive biological objects are suspended in water in order to support and protect the sensitive biological objects, such as for short term storage, or when transporting between processing stations.

The so-called fish pumps are now increasingly being applied in aquaculture in transporting fish from one location to another (Lemmon, 1977, US4193737A; Lindgren 2004, US7462016B2). The method of transport can be called a floatation method where the fish is being carried as floating object in large amount of water through a wide diameter pipe. This technology is now also being increasingly used for transporting fish parts during processing, such as fish intestines, livers and other but also this is used for transport of other types of food components (Halse, & Falkjar, 2014. US9717258B2). The distances of transport are usually not very long, but can be up to 1000 m or more. This type of transport is considered safe and economical and prevents damage to the living fish but is also considered gentle for other transport, such as for fish components. The pipes are wide, or typically 20 to 50 cm in diameter and the water is moved by suction (vacuum) or by air pulses. The materials used in such pipelines can be of different materials, such as plastics or metals.

The aquaculture industry is highly sensitive for infections and cross-contamination of microbes or parasites and must be operated under strict cleanliness and the best possible hygienic conditions (Manual of tests for aquatic animals, 2009). Aquaculture in the open water is now generally operated in clusters of cages, the clusters being located far from each other and they also go through rest-periods, both in order to diminish environmental load as well as to clean up infections and parasites that may have come up in one site before next lot of fish is being grown there. Each lot can be grown from 1-2 years in same location and then all is taken out before next lot is put in and the process is restarted. This and other measures are now taken to prevent cross contamination between lots. Furthermore the best practice operators use separate, boats and all separate gear, clothing, boots and tools for each cage cluster and if same persons are to service the different clusters they must change clothes that are all kept in separate rooms that are physically kept away from each other by significant distance, often by 50-100 m distance from the service room of another cage cluster. Even in many cases separate boats are used to service each cluster. This shows how much emphasis is put on prevention of possible transfer and dispersal of pests and disease.

Any measures of this kind are therefore very costly compared to if same equipment could be safely cleaned and disinfected so they could be safely used for more clusters and lots. Since fish must often be transferred from one location to another within the operational facilities and areas, including between cages or to slaughter and processing house, it is imperative for preventing any cross contamination that otherwise could result from such transfers.

Also since the fish-pump type transfer apparatus is now increasingly being used for the transport of other wild fish, seafood, such as shrimp, as well as offal, livers and different parts that need to be transported from one place to another within the operational facilities, the pumping apparatus must be thoroughly cleaned and disinfected as well to lower the general microbial loads and to prevent contamination by pathogenic microbes, such as *Listeria.*

The use of fog systems for disinfection different kind of facilities, pipes and tanks is well known in the art (Agmont e Silva, US 2017/0173199). Such fog disinfection systems are known to be very economical and efficient, compared to direct wash or filling with disinfecting solutions. US4537749 discloses a system for sterilizing open-topped containers by vaporizing a sterilizing agent in the vicinity of the container. A conveyor is used to bring the container in proximity to a nozzle and a heater element, and a sterilizing agent from the nozzle is delivered to the heater element where it undergoes thermal decomposition and condenses on the surfaces of the container.

However, there is a special problem in applying fog disinfection to wide diameter pipes, and more so when the wide diameter pipe systems become very long, such as when extending for several hundred meters. Wide diameter pipelines as discussed here also bear similarities to ventilation ducts that also may need to be disinfected, for example in facilities where a highly contagious biological agents such as the COVID 19 virus, have occurred. Such wide pipe systems are often operated by, or equipped with air pumps, so that in principle the disinfecting fog could be blown into one end of the wide pipe system, and it might be expected that turbulent air flowing through the pipes would be able to carry the disinfecting fine droplets for long distances in the pipes. Also because of turbulence it might be expected that the very fine droplets with disinfectant would therefore touch all parts of the pipes so that the whole pipeline system would be sufficiently disinfected.

However, the above assumptions do not hold, since once the fine droplets have been blown out of the fog nozzles, several things happen that can prevent the effective and complete action of the disinfecting fog to do its job. As soon as the droplets start touching any surface they stay there or condense and therefore lowering the concentration of remaining airborne droplets. Furthermore the airborne droplet when touching each other, stay together and become gradually larger, and therefore heavier and will fall down at a faster rate. For the airflow to remain turbulent an extremely strong airflow would be required and even if the air is flowing at a fast rate, it will still tend to go into a laminar flow pattern in long and wide pipes. Also the droplets will still, on average, keep falling down, even in turbulent airflow, but faster in laminar flow. Due to these considerations the effective disinfection will only work for a certain distance into the pipes, but also after a relatively short distance from the fog source, the concentration of the fog droplets touching the ceiling of the pipes will become much lower than those touching the sides and the floor. Such behavior and different effectiveness of fog systems in wide pipes and tunnels have been studied by others (Crosfield, et al., 2009). Wide and long pipelines will also be subject to air flow that is influenced and/or may be dominated by the natural air flow in the surroundings, such as wind strength and direction or suction effects created from different temperatures around different parts of the pipeline.

### SUMMARY

The present invention seeks to overcome and/or mitigate the above described deficiencies of the prior art.

In the present invention we describe the characteristics of an apparatus and method for sanitizing or disinfecting of closed spaces such that when the space is in use and filled with sensitive biological objects, such as live fish, the atomizing unit (e.g. nozzles) needed for making and providing disinfecting fog, is kept outside the space, but can be moved inside when the space has been emptied and when disinfection is needed.

Accordingly, there is provided a sanitizing apparatus that during use can have at least two positions. In a first position, the apparatus can deliver a sanitizing or disinfecting fluid such as a fog or mist into a space in need of sanitizing (a receiving space) by means of an atomizing unit within the apparatus. In a second position, the atomizing unit is isolated from the space in need of sanitizing by means of a sealing mechanism that prevents potential contaminating material or liquid from entering the sanitizing apparatus.

The apparatus thus comprises a housing, defining an enclosed interior space and having an opening for connection to an opening in a receiving space such as a shaft, pipe or a container (a receiving space). The receiving space can be a closed or partially closed structure. The receiving space can further comprise a generally closed structure having one or more slots or openings for entry and/or exit of items, such as food items. Such openings in the receiving space can be closable in a reversible manner by doors, lids or the like. The apparatus further comprises at least one atomizing unit that is suitably disposed within the housing. The atomizing unit serves the role of generating and delivering fine spray, fog or mist into a receiving space, the at least one nozzle being operatively connected to a moving mechanism. Further the apparatus comprises a moving mechanism, positioned within the housing, the moving mechanism having a first position and a second position, wherein when the moving mechanism is in the first position, the at least one atomizing unit is positioned within the housing and when the moving mechanism is in the second position, the at least one atomizing unit is at least partially positioned outside the housing, and wherein movement from the first to the second position comprises movement of the at least one atomizing unit towards the opening on the housing. To isolate the interior of the housing when in the first position, the apparatus also comprises at least one sealing member, operatively connected to the moving mechanism and adapted to sealably close the housing when the housing is connected to a receiving space and the moving mechanism is in the first position.

The apparatus housing can have a generally elongate shape. The apparatus housing can have a generally tubular shape, or the housing can be elongate having a rectangular cross-section, i.e. having four elongate walls assembled in a rectangular fashion.

The at least one atomizing unit and the at least one sealing member are preferably supported by the moving mechanism, so that the moving mechanism, when moving from a first to a second position, actively is engaged with and moves the atomizing unit and the sealing member from their respective first to respective second position. Thereby, the apparatus, when connected to a receiving space is operable so that the at least one atomizing unit can move from a first position, wherein the at least one atomizing unit is positioned within the housing, with fluid being prevented from entering said housing from the receiving space by said at least one sealing member, to a second position, wherein the atomizing unit is at least partially positioned outside the housing, thereby entering said receiving space and allowing fluid in the form of fog, mist or fine spray that is ejected from said atomizing unit to enter said receiving space.

The opening on the housing can be adapted so that it can engage with, or encircle, an opening on a receiving space, i.e. an opening in a wall of a receiving pipe, shaft, container or the like.

In the present context the term "container" refers to any object for holding or transporting items, including but not limited to boxes, holders, receptacles and/or vessels for holding or transporting food items, including fish, meat or agricultural products. The engaging can include a reversible/releasable connection to the receiving space, by means of any known mechanism known in the art, such as clamping, bolting, screwing etc. The engaging can also comprise welding the sanitizing apparatus to the receiving space. The engaging can be done via a flange or connector on the receiving space, that extends from or encircles an opening in the receiving space. This way, a modular flange or connector can be used to attach the sanitizing apparatus to a receiving space at any desired position thereon. The flange or connector ensures a sealable connection to the apparatus, thus allowing the opening into the receiving space to be non-modular in size and/or shape. Further, irregular shape of the outer surface of the receiving space, for example when in form of a pipe or shaft with variable curvature, a modular flange or connector that encircles or covers an opening into the receiving space can allow connection of the apparatus without modifications thereof to ensure secure connection to the receiving space.

The moving mechanism serves the role of moving the atomizing unit between a position wherein the atomizing unit is at least partially within the receiving space (second position) to a position outside the receiving space and within the housing (first position). In the first position, the sealing mechanism closes off the inside of the housing from the receiving space. This prevents liquid or biological material (e.g. food products or food parts such as fish or fish parts) from entering the housing when the receiving space is in use, e.g. when food products such as fish are actively being transported and/or stored within the receiving space.

The moving mechanism can comprise or consist of one or more rod or piston that can move in direction that is perpendicular to a tangent plane of the surface to which the apparatus is connected. This way, a movement from a first to a second position takes the piston towards and partially into the receiving space, thereby moving the atomizing unit and the sealing member, that are both connected to the piston, towards the receiving space. When so provided, the housing can be in the form of an elongate structure, with one or more rod or piston within the housing providing the movement mechanism, which provides movement along the axis of the elongate housing, perpendicular to a tangent plane of the receiving space.

Movement of the moving mechanism from the first to the second position can comprise an axial movement of the rod or piston towards said receiving space, along a longidudinal axis of the housing.

The sealing member can comprise or consist of a sealing plate that is adapted to engage with the housing when the moving mechanism is in the first position, providing a seal between the housing and the receiving space, preventing liquid or biological material from entering said housing from said receiving space. The sealing plate thus engages with the housing in the first position to close off the housing from its surroundings, thereby preventing contaminating material from entering the housing. In this position, the atomizing unit is positioned within the housing, as it is positined proximally to the sealing plate (i.e. closer to the interior of the housing). The sealing plate can be flat or it can have a curvature that is designed to match a curvature in the wall of the receiving space at the location of the opening to which the moving mechanism is attached. This way, as the moving mechanism is in the first position, a seal between the atomizing unit and the receiving space can be ensured. The sealing can comprise a sealant, such as a EPDM or other rubber sealant, or its surface to ensure that a seal be formed when the moving mechamism is in the first position, touching the housing to provide a sealing connection therebetween to prevent fluid from entering the housing.

The housing can have a sealing support to which the sealing member (e.g., sealing plate) is configured to engage to provide a fluid-proof connection therebetween. The sealing support can be in the form of a surface having a sealant (a rubber sealant or other known sealant in the art) to provide a leak-proof (fluid-proof) connection with the sealing plate. For example the sealing support can be in the form of a plate that traverses the housing (e.g. an elongate housing) at an angle to its longitudinal axis (e.g. at a 90° angle to the longitudinal axis), the plate having an opening therein to allow the movement mechanism to move through the plate, the plate receiving the sealing plate of the moving mechanism so that the sealing plate and the sealing support (in the form of a plate) form a sealing connection therebetween.

The atomizing unit can be fully inside the receiving space in the second position, i.e. movement from the first to the second position takes the atomizing unit from a position where it is positioned within the housing to a position where it is positioned outside the housing and inside the receiving space to which the apparatus is connected. The atomizing unit can also be partially located within the receiving space in the second position, i.e. a part of the atomizing unit (e.g. a nozzle) is within the receiving space and a portion of the atomizing unit is within the housing. It can however be preferable that the opening or nozzle through which the atomized fluid (fog or mist) is delivered is fully within the receiving space in the second position, to ensure that delivered atomized fluid is delivered into the receiving space.

The atomizing unit can be conveniently mounted on an inwardly side of the sealing member (e.g. the sealing plate), i.e. on the side of the sealing member that faces the interior of the housing. Thereby, when the sealing member (e.g. sealing plate) is engaged with the housing to generate a seal between the housing and the sealing member, the atomizing unit will be positioned within the housing, and thereby isolated from the receiving space to which the apparatus is connected, so that liquid or biological material is prevented from entering the housing.

The apparatus can be provided as a double lid system where the atomizing unit is positioned between two lids so that when the receiving space is in use, e.g. filled with water and/or food material being transported (e.g., fish), an inner lid is connected to and sealably closes the receiving space to which the apparatus is connected, and when disinfection of the receiving space is needed the double lid system moves inwardly with an outer lid sealably closing the receiving. In this second position, the atomizing unit needed for providing the disinfecting fog is positioned inside the receiving space so that sanitizing fog or mist generated by the atomizing unit can be delivered into the receiving space. Since the outer lid closes the wall, fog or mist delivered by the atomizing unit stays inside the treated receiving space.

The apparatus can further comprise a deflecting member, which purpose is to deflect debris or liquid away from the apparatus. The deflecting member can be in the form of a deflecting plate that is connected to the rod or piston at a distal end thereof (i.e. distal to the sealing plate), wherein the deflecting plate is configured so that when the apparatus is connected to a receiving space the deflecting plate is positioned within the receiving opening on said receiving space. The deflecting plate can be at least partially flush with an inner wall of the receiving space, flanking the receiving opening thereof. The deflecting plate can preferably be configured to be at an angle with respect so the sealing plate. Thereby the deflecting plate can be configured to be at an angle with respect to the flow of liquid and/or food items transported in a pipe or duct to which the apparatus is connected. By having the deflecting plate at an angle with respect to such flow of liquid and/or food items, any liquid, debris or food items that are carried by the flow within the piping, e.g. by liquid flowing within the pipin, will be deflected away from the apparatus, thereby minimizing or preventing such liquid or food items from entering the housing.

The apparatus can further comprise piping means, for delivering liquid and/or gas into said atomizing unit. Such piping are known in the art. For example, the piping means can comprise a first pipe, for delivering sanitizing fluid into the atomizing unit, and a second pipe, for delivering gas or air into the atomizing unit.

The atomizing unit can comprise any suitable atomizing apparatus that can generate a fog or mist from a liquid. Any such atomizing apparatus is compatible with the sanitizing apparatus of the invention. Examplary atomizing units include atomizing nozzles or atomizing discs known in the art. Atomizing units in general comprise a housing having an aperture through which fine droplets exit to generate a stram of fog or mist. The housing can have an ultrasonic atomizer within the housing proximate the aperture. Alternatively the housing can comprise or consist of an atomizing nozzle, into which a pressurized gas (such as air) stream is injected together with a liquid, thereby generating fine droplets. The atomizing unit is supplied fluid and/or air as required for its function.

The apparatus or system comprising the apparatus can further comprise at least one sanitizing tank for holding a sanitizing fluid and at least one pressurized gas tank, for holding pressurized gas and/or air. The apparatus can additionally comprise one or more controller, for controlling fluid and/or gas delivery into said at least one atomizing unit. The controller can include and/or allow remote electronic control of the apparatus, including pumps, valves and motors, and reporting to and from the apparatus. The controller can further receive input from sensors within the system, such as temperature and humidity sensor, pressure sensors and volume sensors.

Other components of the apparatus can be included, for example units that can control the strength of disinfectant, mixing of disinfectant and water, and/or disinfection time.

The moving mechanism can be driven pneumatically, i.e. by pressured air that can be used for driving movement of the mechanism including the atomizing unit (e.g., atomizing nozzle) from the first to the second position. The mechanism can also be powered by one or more electric motor, or by other suitable means known in the art.

To secure that all sections of a receiving space being treated will be well sanitized or disinfected, there can be means for providing additional air flow in the apparatus. This means can be in form of a nozzle for delivering supplementary air, that is preferably mounted on the sealing plate. This means of supplementary air flow can alternatively be in the form of an opening or hole on a pipe that is connected to the sealing plate. Through this additional air delivery means, an increase in the flow rate of the disinfecting fog can be achieved. Also, the additional air flow can adjust the amount direction of the air that is needed to carry the fog or mist effectively for long distances inside a receiving space, for example when provided as an elongate pipeline. Further, the natural air flow can be taken into consideration during operation of the apparatus and the disinfection process as described here can therefore be controlled and operated such that the disinfecting fog or mist can be directed to flow in the same direction as the natural air flow within the receiving space.

The apparatus can be made by using any number of different materials available to those skilled in the art, or combination of different types of materials. Such materials include, but are not limited to, metals (including stainless steel) and various synthetic materials such plastics.

In certain embodiments of the invention the apparatus can be controlled via a preprogrammed computer and software or as a coordinated task involving valves and switches coordinated beforehand and running in a prefixed/preprogrammed manner. Likewise the measurement of temperature, levels of water and active substances used for disinfection and pesticide control and time of disinfection can be based on preprogrammed measurements in the system or through exact measurements in each space and pipelines. Such measurements of the results of the disinfection and operation can be fed back to the control unit of the system to intensify and increase/decrease concentration of active substance in the fog, the droplet size of the fog, frequency of the delivery as well as time or intensity of the treatment.

The atomizing unit or nozzle can be fed from a control unit that contains at least two containers for water and concentrated disinfectant solution. The container for water can be pressurized to deliver water through a pressure equalizer going through the pipes leading into the apparatus, where the atomizing unit (e.g. provided as nozzles) produces fog or mist that can contain droplets with diameter of about 10 µm that spread through the space to be treated.

The invention also relates to the use of an apparatus that makes disinfectant from aqueous solutions containing different concentrations of dissolved materials made of different active substances well known in the art and composed of a mixture of active substances and liquid of different proportions.

The invention further relates to a system that includes a transport and/or storage container having internal surfaces in need of sanitizing; and at least one sanitizing apparatus connected to a receiving opening on said food transport and/or storate space, wherein said sanitizing apparatus comprises a housing, at least one atomizing unit within said housing, for delivering a sanitizing fluid in the form of mist, fog or fine spray into said food transport and/or storage space; a moving mechanism connected to said at least one atomizing unit, for moving said at least one atomizing unit from a first position, wherein the at least atomizing unit is positioned within the housing, to a second position, wherein the at least one atomizing unit is positioned at least partially within the food transport and/or storage space; and at least one sealing mechanism, for preventing fluid and/or food items within the food transport and/or storage space from entering the sanitizing apparatus when the moving mechanism is in the first position.

The invention also relates to a method of cleaning and/or sanitizing internal surfaces of a space within a structure, the method comprising steps of (i) providing a sanitizing apparatus contained within a housing; (ii) attaching the sanitizing apparatus to the structure, so that the sanitizing apparatus encircles and/or covers an opening in the structure, thereby allowing fluid delivered by the sanitizing apparatus to enter the space; and (iii) delivering a sanitizing fluid from the sanitizing apparatus into said space; wherein the sanitizing apparatus comprises an atomizing unit, for generating and delivering atomized fluid in the form of mist, fog or fine spray, and a moving mechanism, for moving the atomizing unit from a first to a second position, wherein in a first position, the atomizing unit is positioned outside said space and within said housing so that fluid is prevented from entering the housing from said space by a sealing member of said moving mechanism that prevents fluid from said space to enter said housing, and a second position, wherein the atomizing unit is positioned at least in part within said space, thereby allowing spray or atomized fluid delivered by the atomizing unit to enter said space, thereby sanitizing internal surfaces thereof.

The structure can be any structure in need of controlled disinfection and/or sanitizing, such as food transport or food storage system, and air ventilation ducts in buildings which are used for ventilation, heating or air conditioning. The structure can be a closed structure, i.e. a structure that contains surfaces and/or apparati within a housing. The closed structure can be partially open, i.e. it can contain open ends when provided as a pipe or pipeline, or other openings required or useful for its functioning.

In some embodiments, the apparatus can contain two sealing plates (two lids) that are provided such that in the first position, an inner sealing plate connects to the receiving space, thereby preventing fluid from entering the apparatus from the receiving space, and in the second position an outer sealing plate connects to the receiving space to also prevent fluid from entering the apparatus from the receiving space. This way, a double-lid type apparatus can be provided. The inner and out sealing plates (or lids) can be of substantially equal dimensions. The inner and outer sealing plates can be adapted to align with the wall of the receiving space, providing a smooth inner surface of the wall when the apparatus is in the first and second position. In the second position, the atomizing unit is positioned within the receiving space, allowing for delivery of atomized fluid into the space.

In one such embodiment, of the invention the double lid system can be attached to a receiving space by a hinge on the wall of the receiving space. By rotation around the hinge so provided, the apparatus can move from a first position, where a first lid or sealing plate closes off the receiving space with the atomizing unit being positioned outside the receiving space, to a second position, where a second lid or sealing plate closes the receiving space, with the atomizing unit now being positioned within the receiving space.

In some embodiments the apparatus comprises one or more additional pipes that are connected to the sealing plate, or at least to the first sealing plate when the apparatus contains two sealing plates. Such additional pipes can be used to provide additional air or gas to control and direct airflow from the apparatus into the receiving space, thereby enhancing the delivery and distribution of the sanitizing fog or mist delivered by the atomizing unit.

The invention uses known chemical and physical principles to combine in a new way a set of actions and design of an apparatus that makes the actions possible and therefore useful and commercially viable. The invention is therefore an efficient method and is expected to be useful and consequently to have large potential for industrial application and commercial use.

Additional features and advantages of the present invention are described in, and will be apparent from, the following detailed description of embodiments of the invention.

### BRIEF DESCRIPTION OF THE DRAWINGS

FIG. 1 shows a side view of a sanitizing apparatus in accordance with the invention in a closed position.
FIG. 2 shows a side view of a sanitizing apparatus in accordance with the invention in an open position.
FIG. 3 shows a front view of a sanitizing apparatus in accordance with the invention in a closed position.
FIG. 4 shows a front view of a sanitizing apparatus in accordance with the invention in an open position.
FIG. 5 shows a close-up front view of a sanitizing apparatus, showing tubing for supplying the apparatus with liquid and air.
FIG. 6 shows a front view of one alternative sanitizing apparatus in a closed position (A) and open position (B).
FIG. 7 shows a front view of a second alternative sanitizing apparatus in a closed position (A) and open position (B).
FIG. 8 shows a front view of a third alternative sanitizing apparatus in a closed position (A) and open position (B).

### DESCRIPTION

The current invention relates to new ways to use highly desirable sanitizing or disinfection properties of fogging systems for pipelines, pumps and tanks, such as for so-called fish pumps and associated tanks and pipelines. The invention is also applicable to disinfection of other types of food transport pipes, pipelines, pumps or tanks, or for ventilation ducts.

As described herein, the invention is particularly used for rapid disinfection of large food transport systems, such as pipelines and pumps, such as for fish pumps and associated pipelines, the system having an external equipment (sanitizing apparatus) with means to open and close off the interior of the equipment, that includes a nozzle holding plate or lid. The external equipment is connected to a receiving space, i.e. a food transport system, generating sanitizing fog or mist within the receiving space. The apparatus may also contain additional means of dispersing the sanitizing fog inside the space.

The sanitizing apparatus is a multifunctional device that can be made to fit into small spaces or attached to the exterior wall of different types of closed spaces (receiving spaces) such as fish or meat pipelines and ventilation ducts.

The apparatus and method described herein therefore makes frequent disinfection of various closed receiving spaces possible between uses, without any parts of the disinfection apparatus staying inside or protruding into the receiving space when the receiving space in use. This feature is important for many applications, especially where sensitive and soft biological materials, including life fish, is being supported or transferred in such spaces or pipes.

Due to the sensitive nature of biological tissues and life organisms, such as fish, no disinfection can take place while it is in use, but on the other hand, a very thorough and frequent disinfection is needed to prevent growth and crossover contamination of harmful microorganisms.

A disinfection process includes introduction of a sanitizing fog inside the receiving space, i.e. tanks and/or pipelines. One or more atomizing unit, for example fog nozzles, can be mounted onto a sealing plate or lid, such that when the lid is in a closed position, the lid closes off the interior of the sanitizing apparatus, and preferably provides a smooth or nearly smooth inner surface of the tank or pipeline interior. This design secures that fish or other soft food products being transported are not affected, scratched, or hooked on to the sanitizing apparatus when the sanitizing apparatus is not in use and in a closed position. When the pipelines have been emptied between use, the sanitizing apparatus moves to a second open position so that sanitizing fog or mist can be delivered into the receiving space (tank or pipeline). Further, the direction of the flow of mist or fog can be controlled, thereby allowing for a quick and efficient disinfection of wide or elongate spaces such as fish pumps, pipelines and ventilation ducts.

The apparatus and method described, therefore makes disinfection of food (e.g., fish) pumps, tanks and pipelines, fast, easy and safe. The whole sanitizing or disinfection process can be automatically operated and preprogrammed as needed. This will lead to lower cleaning costs and prevent cross-contamination during transport of food products, such as life fish in aquaculture, and between different lots of fish parts or other food products that are being pumped and transported (for example by water in floating mechanism) in such pipeline systems.

Embodiments of the invention that are adapted for rapid disinfection of food transport or storage systems, including wide or elongate pipelines and pumps, such as fish pumps and associated pipelines, will now be described with reference to the drawings and figures provided.

Unless defined otherwise, all technical terms used herein have the same meaning as commonly understood by one of ordinary skill in the art to which the invention belongs. Although any methods and materials similar or equivalent to those described herein can be used in the practice or testing of the present invention, exemplary methods and materials are now described.

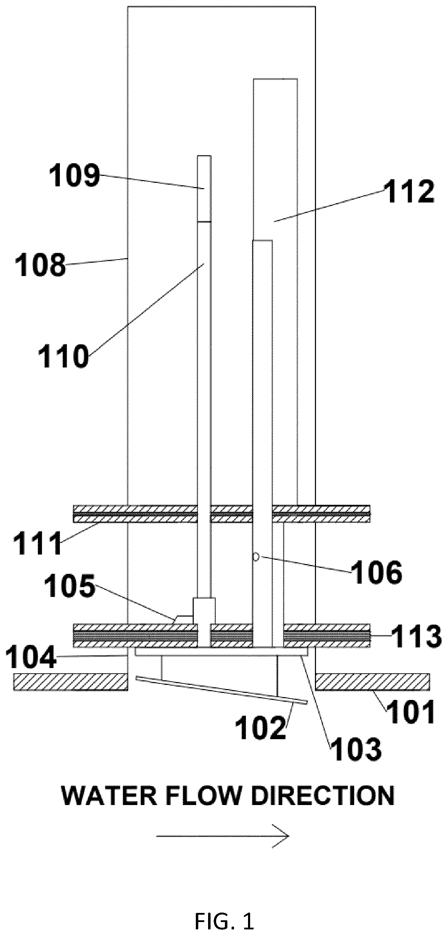

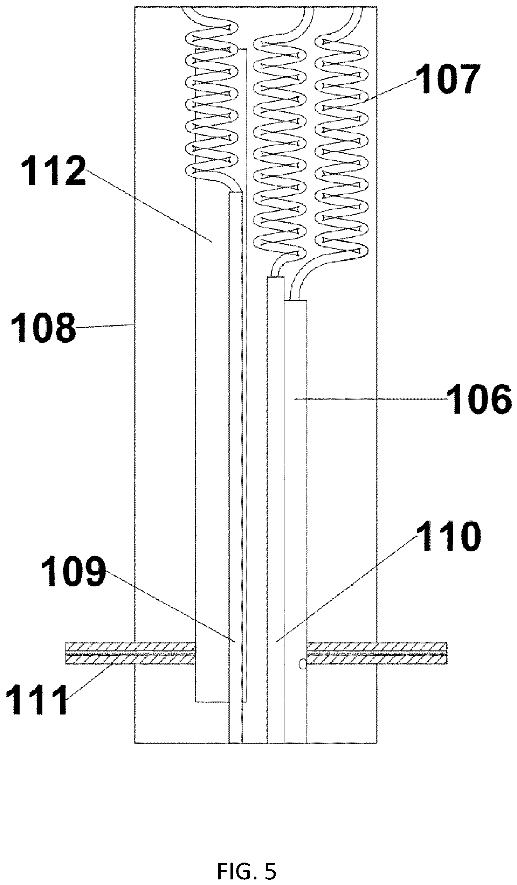

In Figures 1-8 are shown examples of embodiments of a sanitizing apparatus in accordance with the invention. The apparatus can generally comprise a housing having an opening at one end, for attachment to a complimentary opening in a receiving space, i.e. a space in need of sanitizing or disinfection, such as a food processing or food transport and/or storage system, e.g. fish transport pipe, ventilation ducts etc. As will be appreciated, the sanitizing apparatus can be used with any such system or space. In general, a single sanitizing apparatus can be used to disinfect different portions of a system, by connection to a respective opening in the system. Alternatively, there can be a plurality of sanitizing apparatus units connected to a system at various portions thereof, to thereby allow for sanititizing of different sections of the system as needed. In particular, when the sanitizing apparatus is attached to a system by a reversible attachment, the number of sanitizing apparatus units used to sanitizing a particular space can be varied as needed to achieve sufficient distribution or coverage of the sanitizing fog within the space.

Turning to FIG. 1, a side view of a sanitizing apparatus in accordance with the invention is shown. The apparatus comprises a housing **108** that has been attached to a receiving by connection to a wall **101** thereof. In the embodiment shown, there is a flange **104** attached to the wall **101.** Connection between the sanitizing apparatus and the receiving space can therefore be achieved by connection to flange **104.** An advantage of such a configuration is that flange **104** can be of fixed dimensions, i.e. size and/or shape. This way, a single type of sanitizing unit with fixed dimensions of the housing **108** can be attached at a variety of positions in any convenient location on a receiving space. The connection can be made at surfaces that are flat or bent, concave or convex, since the flange **104** provides uniform dimensions for attachment to the receiving space.

As will be appreciated the housing **108** can also be connected directly to a receiving space by direct connection to a side wall **101** thereof, to encircle or cover an opening in the wall **101,** i.e. the connection to the side wall **101** is achieved without the use of a flange **104.**

The receiving space can be a fish transportation pipe. The dimensions of such a pipe can in general range from about 100 mm in diameter up to 1-2 meters in dimeter or even more. In particular, when provided with a flange, the curvature or outer shape of the receiving space (e.g., a fish transport pipe) does not impede the connection of the apparatus to the receiving space. A fish transporation pipe can be of any length, from 1 meter or less to 10 meters or more. If needed, multiple santizing apparati can be connected to a single receiving space, e.g. along the length of a transporation pipe.

A sealing plate **103** is connected to a driving mechanism **112,** that provides force for moving the sealing plate in a linear fashion away from or towards a receiving space, driven by pneumatic air cylinder. The sealing plate is in the position shown touching sealing support **113** to provide a seal between the receiving space and the inside of the housing **108,** and thereby close off the housing **108.** This way, the inside of the housing **108** is isolated from the receiving space to which the sanitizing apparatus is connected, so that for example water or debris flowing within the apparatus (e.g., when in the form of a fish transportation pipe) can not enter the housing. The sealing support **113** can comprise a sealant, such as a EPDM or other rubber sealant. Such sealant serves two purposes, i.e. it provides a seal between flange **104** and housing **108** and it also provides a seal between the sealing plate **103** and the sealing support **113.**

A nozzle **105,** for delivering a fog or mist, is mounted on the sealing plate **103,** on an internal side therof, i.e. facing the inside of the housing. The nozzle is partially hidden in this side view. When the moving mechanism is in a closed position and the sealing plate is touching sealing support **113** of the housing, the nozzle **105** is within the housing, as shown in this Figure. Pipes **109, 110** provide liquid and gas/air to the nozzle, respectively. A secondary air supply pipe **106** is connected to the sealing plate. A hole or opening in the pipe **106** can provide additional air in the direction of delivery of the mist or fog, to enhance the distribution of the sanitizing fog or mist within the receiving space.

A deflection plate **102** is connected to the sealing plate on the outside thereof, i.e. facing away from the plate and towards the receiving space. The deflection plate **102** is slanted towards a flow of direction within the receiving space (e.g. fish transportation pipe), i.e. the plate is closer to the sealing plate **103** at one end (front end) than the opposite end (back end), i.e. a back-to-front direction of the sealing plate is generally opposite the flow of direction within the receiving space. The purpose of the deflection plate is to deflect moving material within the receiving space from being damaged by by the sanitizing apparatus, by preventing entering the cleft or cavity generated between the sealing plate **103** and the adjacent side walls **101** of the receiving space. This way, damage to transported items within the receiving space can be avoided or minimized. For example, when the receiving space is a fish transportation pipe, fish that is being transported could be damaged if hitting or entering the cleft or cavity at speed. The deflection plate prevents this from occurring, providing for safe functioning of the transporation pipe when in use, with the moving mechanism of the sanitizing apparatus in a closed position.

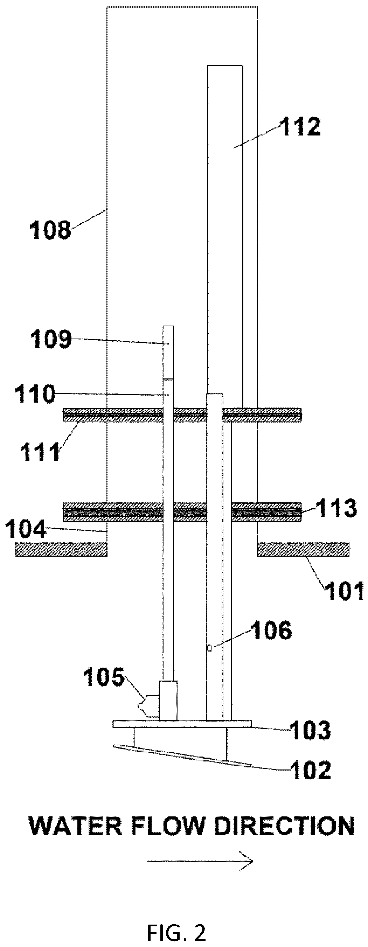

In FIG. 2, the sanitizing apparatus is shown with the moving mechanism being in an open position. In this position, sealing plate **103** has moved away from the plate **113** in the housing, with the result that nozzle **105** is positioned within the receiving space. In this position, the sanitizing apparatus is operated, with sanitizing fog or mist being generated and delivered by the nozzle **105.** In the embodiment shown, the nozzle will deliver mist or fog in direction that is generally against the flow of direction of the reciving space, when in use. However, the nozzle can alternatively be oriented in any particular fashion to maximize the distribution of fog or mist within the receiving space. The hole or opening in the optional pipe **106** can also be used to provide additional air in the direction of delivery of the mist or fog, to enhance the distribution of the sanitizing fog or mist within the receiving space.

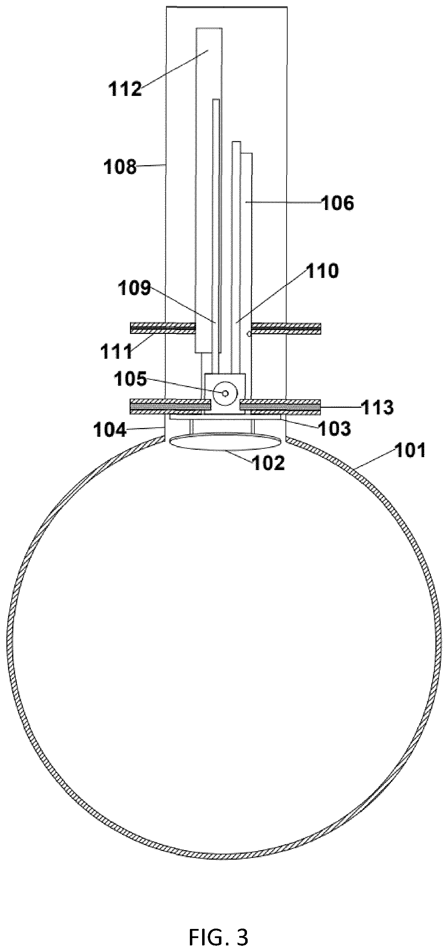

In FIG. 3 a front view of the sanitizing apparatus in a closed position, attached to a receiving space **101** in the form of a transportation pipe, is shown. In this view, an advantage of the deflecting plate **102** becomes apparent. The deflecting plate **102** is slanted towards the incoming flow within the transporation pipe, thereby deflecting the items away from the sanitizing apparatus and thereby the cleft or cavity between the sanitizing apparatus and the interior of the transportation pipe.

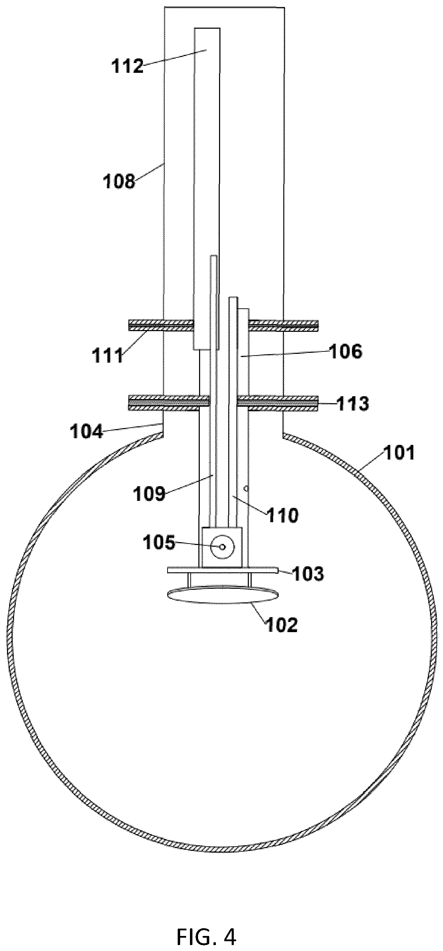

A front view of the apparatus as shown in FIG. 3 in an open position is shown in FIG. 4. Here, the sealing plate **103,** together with attached nozzle **105,** has moved towards the interior of the transporation pipe **101.** In this position, sanitizing fog or mist can be delivered through the nozzle **105** to sanitize and/or disinfect the transportation pipe **101.**

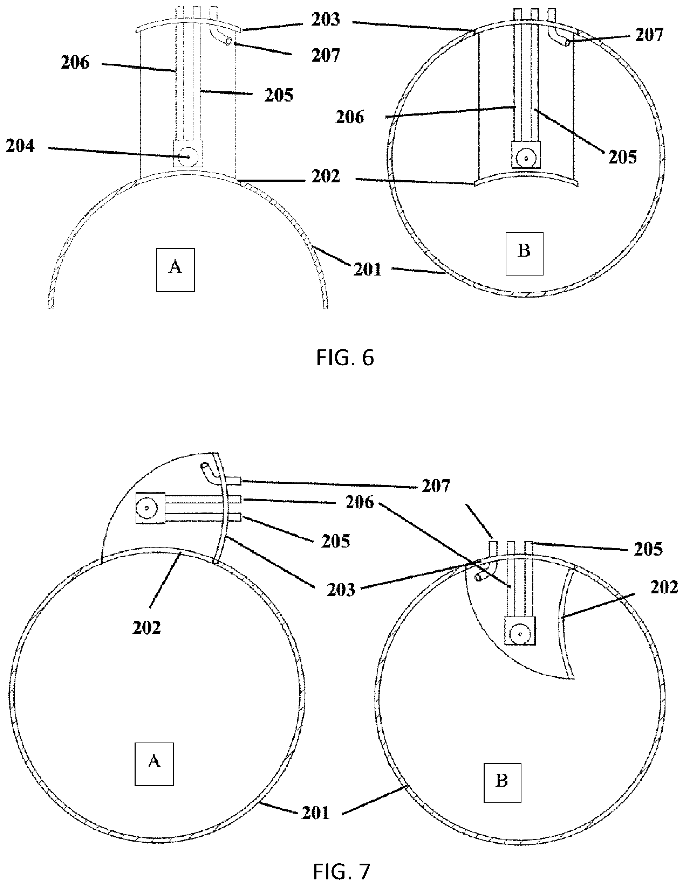

Nozzle **105** receives air and liquid (i.e. sanitizing liquid) through pipes **109, 110.** Supplementary air can be delivered through pipe **106** that is also attached to the sealing plate. As shown in FIG. 6, air and/or gas into these pipes is supplied by tubing **107,** that should be flexible to be able to accommodate the movement of the pipes during operation of the apparatus. The tubing can therefore either be a soft tubing and/or a spiral-shaped tubing that provides flexibility with respect to the inward and outward movement of the pipes **106, 109 ,110.** The tubing is connected to external sources of pressurized air and liquid (not shown).

In FIGs 6 - 8 there are shown three further examples of a sanitizing apparatus in accordance with the invention. In these embodiments, the apparatus is provided as a double lid system that can be positioned in a wall of a closed receiving space to be disinfected. In these double lid embodiments, the internal wall of the receiving space is closed off by sealing plates that are adapted to close off the opening in the wall of the receiving space to which the sanitizing apparatus is connected, providing a flush sealing connection with the wall that follows the contours of the wall surrounding the opening.

FIG 6 shows a front view of an embodiment where the housing of the sanitizing apparatus contains a double lid **202, 203** with nozzle **204** being moved in and out by a jack action. The double lid is attached to the wall **201** of the receiving space to be disinfected, with inner sealing plate or lid **202** that is flush against the wall, following its contour and thereby closes the wall in a seamless manner. The mechanism can move from a first position, shown in (A) to a second position, shown in (B). In the first position, an inner sealing plate or lid is in a closed position with the wall **201,** isolating the housing from the receiving space, here shown as a pipe structure, for example a pipe for transporting fish. For disinfecting the apparatus moves inwardly to a second position, such that the outer sealing plate or lid **203** closes the wall in a same manner as the inner sealing plate, with the disinfecting nozzle **204** now positioned within the receiving pace. The inner and outer sealing plates (or lids) have substantially the same dimensions, so that they can both provide a secure closure of the opening in the wall **201,** preventing liquid or debris from escaping from the pipe. The nozzle **204** is connected to pipes for delivery of pressured air **205** and disinfecting fluid **206.** A separate pipe **207** is provided for the additional air to adjust and control air flow and direction when the apparatus is in use.

In FIG 7 an alternative embodiment is shown, having an inner and outer sealing plate of substantially equal dimension, which are such that the sealing plates can effectively close the opening in the wall 202 to which the apparatus is connected. Here, the apparatus moves from a first to a second position by means of a hinging mechanism, i.e. a hinge is provided around which the apparatus can rotate to move from a first position, where the inner sealing plate closes off the pipe, to a second position, in which the second sealing plate closes off the pipe.

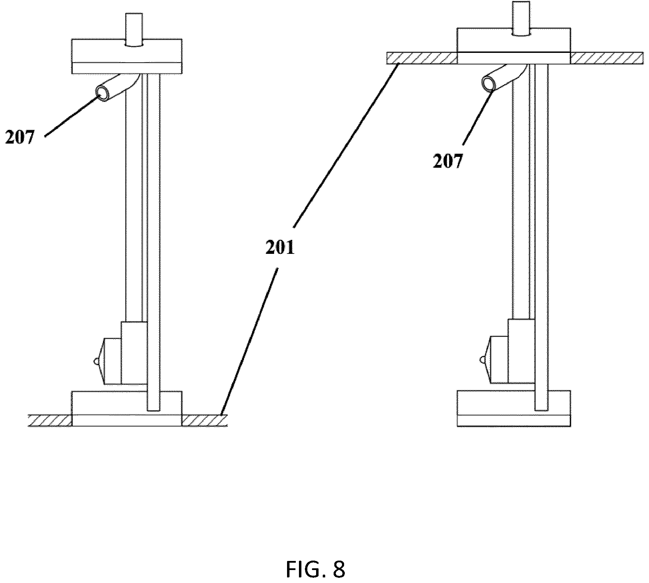

FIG 8 shows another embodiment of a double-lid type apparatus in an open view, i.e. the housing is not shown in this view. Here, the apparatus contains two substantially identical sealing plates or lids that each can provide a leakproof connection to the wall to which the apparatus is connected. The sealing plate in this embodiment is substantially flat, but can otherwise be of any suitable shape, i.e. round, oval or rectangular. In a first position (shown on left, an inner sealing plate closes the receiving space to which the apparatus is connected, with the atomizing unit and other components of the apparatus being positioned outside the receiving space. In the second position (on the right), the outer sealing plate closes the receiving space, with the atomizing unit (nozzle) now positioned within the receiving space. In this position, the apparatus can be operated to deliver disinfecting fog or mist into the receiving space.

As will be apparent from the foregoing, the present invention provides numerous advantages over the prior art:
- Modular unit that can be connected to any desired apparatus or space in need of sanitizing or disinfection
- Multiple units can be connected as desired
- Single type of unit can be used for various applications
- Efficient delivery of sanitizing fog, ensuring coverage of even hard to reach surfaces
- Inner components of apparatus protected from liquid or debris when not in use by means of unique moving mechanism
- Minimal or no manual handling needed, apparatus can be operated automatically
- Time for disinfection minimal, which reduces off-time resulting in cost-savings

As used herein, including in the claims, singular forms of terms are to be construed as also including the plural form and vice versa, unless the context indicates otherwise. Thus, it should be noted that as used herein, the singular forms "a," "an," and "the" include plural references unless the context clearly dictates otherwise.

Throughout the description and claims, the terms "comprise", "including", "having", and "contain" and their variations should be understood as meaning "including but not limited to" and are not intended to exclude other components.

The present invention also covers the exact terms, features, values and ranges etc. in case these terms, features, values and ranges etc. are used in conjunction with terms such as about, around, generally, substantially, essentially, at least etc. (i.e., "about 3" shall also cover exactly 3 or "substantially constant" shall also cover exactly constant).

It will be appreciated that variations to the foregoing embodiments of the invention can be made while still falling within the scope of the the appended claims Features disclosed in the specification, unless stated otherwise, can be replaced by alternative features serving the same, equivalent or similar purpose. Thus, unless stated otherwise, each feature disclosed represents one example of a generic series of equivalent or similar features.

Use of exemplary language, such as "for instance", "such as", "for example" and the like, is merely intended to better illustrate the invention and does not indicate a limitation on the scope of the invention unless so claimed. Any steps described in the specification may be performed in any order or simultaneously, unless the context clearly indicates otherwise. All of the features and/or steps disclosed in the specification can be combined in any combination, except for combinations where at least some of the features and/or steps are mutually exclusive. In particular, preferred features of the invention are applicable to all aspects of the invention and may be used in any combination.

### EXAMPLE 1

Table 1 shows comparison of the properties and effectiveness of the present invention compared with the state of the art of the most currently used methods for disinfecting of spaces such as of transport room of trucks and containers.

**Table 1. Comparison of the disinfection efficiency of a sanitizing apparatus in accordance with the invention compared to current situation of manual cleaning methods.**

| ***Key properties in comparing disinfection methods*** | ***Manual Cleaning*** | ***This invention*** |
|---|---|---|
| Biofilm eradication efficiency | Moderate | High |
| Suitable for difficult-to-access areas | No | Yes |
| Manual handling required | Yes | No |
| Water use per disinfection (L) | High >10 | Low -0.8 |
| Time required (min) | ~30 | ~3 |
| Risk of run-off into environment | Yes | No |
| Biofilm as measured by ATP | High | Low |
| Bacteria on surface - cfu of E. coli | Frequently detected | Not detected |

### EXAMPLE 2

This example demonstrates the use of the sanitizing apparatus of the invention for disinfecting a fish pipe used for transport of living fish as shown in table 2.

**Table 2. Surface count of bacteria per cm² on floor and sidewalls of a 40 cm diameter fish pipe used for transport of live salmon smolt to cage.**

| **Pipe** | **Floor** | | **Wall** | |
|---|---|---|---|---|
| **Sample** | Before D | After D | Before D | After D |
| **Date 1** | 3.000 | 400 | 1.000 | 150 |
| **Date 2** | 1.200 | 50 | 800 | 0 |
| **Date 3** | 1.000 | 0 | 700 | 10 |
| **Average** | 1.733 | 150 | 833 | 53 |

### EXAMPLE 3

This example shows how the use of the sanitizing apparatus of the invention to control fog dispersion and air flow in a pipeline gives optimum distribution and effect of the disinfection fog as well as to minimize the waste of the disinfection fluid which otherwise condenses and runs out on the bottom of the pipeline.

**Table 3. Comparison of the disinfection efficiency of the invention compared to disinfection of a fully closed pipeline with no additional air flow and a very fast air flow as can happen when air flow is uncontrolled by a wind situation or natural air flow in the pipeline. The length of the pipelines were 6 m and fog blown into the pipelines for 120 sec.**

| **Fog distribution in a 6 m long pipeline of a 40 cm diameter** | | | |
|---|---|---|---|
| Air flow | No additional, pipe closed | Fast from natural airflow | Medium, controlled by the invention |
| Time of fog to reach end of pipeline, sec. | 120 | 10 | 50 |
| Condensed disinfection liquid collected, ml | 280^{a} | 50 | 100 |

| **Fog distribution in a 6 m long pipeline of a 20 cm diameter** | | | |
|---|---|---|---|
| Time of fog to reach end of pipeline, sec. | 60 | 20 | 35 |
| Condensed disinfection liquid collected, ml | 350^{a} | 10 | 50 |

The total amount used in each case to make fog was about 500 ml, indicating the ingoing volume. The less volume collected as condensed disinfection liquid coming out of the pipe at the end of the experiment therefor indicated better use and more efficient disinfection.

## Claims

1. Apparatus for delivering sanitizing fluid into a receiving space, the apparatus comprising:
a housing (108) having an opening for connection to an opening in a receiving space wherein the receiving space is selected from a food transport or food storage system, air ventilation duct, a shaft, pipe or a container;
at least one atomizing unit (105) disposed within the housing, for generating and delivering fine spray, fog or mist into the receiving space, the at least one atomizing unit (105) being connected to a moving mechanism;
a moving mechanism (112), within the housing, having a first position and a second position, wherein when the mechanism is in the first position, the at least one atomizing unit is positioned within the housing and when the moving mechanism (112) is in the second position, the at least one atomizing unit (105) is at least partially positioned outside the housing, and wherein movement from the first to the second position comprises movement of the at least one atomizing unit (105) towards the opening on the housing;
at least one sealing member (103) connected to the moving mechanism (112) and adapted to sealably close the housing when the housing is connected to the receiving space and the moving mechanism (112) is in the first position;
wherein the at least one atomizing unit (105) and the at least one sealing member (103) are supported by said moving mechanism; and
wherein the apparatus, when connected to the receiving space is operable so that the at least one atomizing unit (105) can move from a first position, wherein the at least one atomizing unit (105) is positioned within the housing, wherein the at least one sealing member (103) is configured to prevent fluid from the receiving space entering said housing, to a second position, wherein the atomizing unit (105) is at least partially positioned outside the housing, thereby entering said receiving space and allowing fluid in the form of fog, mist or fine spray that is ejected from said atomizing unit (105) to enter said receiving space.

2. The apparatus of claim 1, wherein the moving mechanism comprises at least one rod or piston, wherein the at least one atomizing unit and said at least one sealing member are mounted on said at least one rod or piston, and wherein movement from the first to the second position comprises an axial movement of the rod or piston towards the opening in said housing.

3. The apparatus of any one of the previous claims, wherein the at least one sealing member comprises a sealing plate that is adapted to engage with the housing when the moving mechanism is in the first position, providing a seal between the housing and the receiving space, preventing liquid or biological material from entering said housing from said receiving space.

4. The apparatus of the previous claim, wherein the at least one sealing plate is mounted on said at least one rod or piston.

5. The apparatus of any one of the previous claims 3-4, wherein the at least one atomizing unit is mounted on said sealing plate on an inwardly side thereof with respect to the housing, so that, when the moving mechanism is in the first position, the atomizing unit is positioned within the housing and said sealing plate is in a sealing position that prevents liquid from entering said housing.

6. The apparatus of any one of the preceding claims, further comprising piping means, for delivering liquid and/or gas into said atomizing unit.

7. The apparatus of any one of the previous claims, wherein the atomizing unit comprises an atomizing nozzle or disc.

8. The apparatus of any one of the previous claims 2-7, further comprising at least one deflecting plate that is connected to the distal end of said rod or piston, wherein the deflecting plate is slanted towards a flow of direction within the receiving space so that a front end of the deflecting plate is closer to the sealing plate than an opposite end (back end) of the deflecting plate, and wherein the deflecting plate is configured so that when the apparatus is connected to a receiving space the deflecting plate is positioned within the receiving opening on said receiving space.

9. The apparatus of any one of the previous three claims, wherein the deflecting plate is configured to be at an angle with respect so said sealing plate.

10. The apparatus of any one of the claims 2-9, wherein the sealing member comprises a first and a second sealing plate, wherein the first and second sealing plates are mounted on the rod so that the atomizing unit is positioned in between the first and second sealing plate, and wherein the first and second sealing plates close the housing when the moving mechanism is in the first and second position, respectively.

11. A system comprising
- a food transport and/or storage container having internal surfaces in need of sanitizing; and
- at least one sanitizing apparatus as set forth in any one of the claims 1-10.

12. The apparatus or system of any one of the previous claims, further comprising at least one controller, for controlling fluid and/or gas delivery into said at least one atomizing unit.

13. A method of cleaning and/or sanitizing internal surfaces of a space within a structure, such as a food transport and/or storage container structure, the method comprising steps of:
providing a sanitizing apparatus contained within a housing;
attaching the sanitizing apparatus to the structure, so that the sanitizing apparatus encircles and/or covers an opening in the structure, thereby allowing fluid delivered by the sanitizing apparatus to enter the space; and
delivering a sanitizing fluid from the sanitizing apparatus into said space;
wherein the sanitizing apparatus comprises an atomizing unit, for generating and delivering atomized fluid in the form of mist, fog or fine spray, and a moving mechanism, for moving the atomizing unit from a first to a second position, wherein in a first position, the atomizing is positioned outside said space and within said housing so that fluid is prevented from entering the housing from said space by a sealing member of said moving mechanism that prevents fluid from said space to enter said housing, and a second position, wherein the atomizing unit is positioned at least in part within said space, thereby allowing spray or atomized fluid delivered by the atomizing unit to enter said space, thereby sanitizing internal surfaces thereof.

14. The method of the previous claim, wherein the sanitizing apparatus is an apparatus as set forth in any one of the claims 1 2 -10.

## Patentansprüche

1. Vorrichtung für die Zufuhr von Desinfektionsflüssigkeit in einen Aufnahmeraum, wobei die Vorrichtung Folgendes umfasst:
ein Gehäuse (108) mit einer Öffnung zum Anschluss an eine Öffnung in einem Aufnahmeraum, wobei der Aufnahmeraum aus einem Lebensmitteltransport- oder Lebensmittellagersystem, einem Belüftungskanal, einem Schacht, einem Rohr oder einem Behälter auszuwählen ist;
mindestens eine Zerstäubungseinheit (105), die innerhalb des Gehäuses angeordnet ist, um feinem Sprühnebel, Nebel oder Dunst zu erzeugen und in den Aufnahmeraum abzugeben, wobei die mindestens eine Zerstäubungseinheit (105) mit einem Bewegungsmechanismus verbunden ist;
ein Bewegungsmechanismus (112) innerhalb des Gehäuses, der eine erste Position und eine zweite Position aufweist, wobei, wenn sich der Mechanismus in der ersten Position befindet, die mindestens eine Zerstäubungseinheit innerhalb des Gehäuses positioniert ist, und wenn sich der Bewegungsmechanismus (112) in der zweiten Position befindet, die mindestens eine Zerstäubungseinheit (105) mindestens teilweise außerhalb des Gehäuses positioniert ist, und wobei es sich bei der Bewegung von der ersten in die zweite Position um eine Bewegung der zumindest einen Zerstäubungseinheit (105) in Richtung der Öffnung am Gehäuse handelt;
mindestens ein Dichtungselement (103), das mit dem Bewegungsmechanismus (112) verbunden ist und dazu ausgelegt ist, das Gehäuse dicht zu verschließen, wenn das Gehäuse mit dem Aufnahmeraum verbunden ist und sich der Bewegungsmechanismus (112) in der ersten Position befindet;
wobei die Zerstäubungseinheit (105) und das Dichtungselement (103) von dem Bewegungsmechanismus getragen werden.; und
wobei die Vorrichtung, wenn sie mit dem Aufnahmeraum verbunden ist, so bedienbar ist, dass die mindestens eine Zerstäubungseinheit (105) aus einer ersten Position, in der die mindestens eine Zerstäubungseinheit (105) innerhalb des Gehäuses positioniert ist, wobei das mindestens eine Dichtungselement (103) so konfiguriert ist, dass es verhindert, dass Fluid aus dem Aufnahmeraum in das Gehäuse in eine zweite Position gelangt, in der die Zerstäubungseinheit (105) mindestens teilweise außerhalb des Gehäuses positioniert ist, wodurch sie in den Aufnahmeraum gelangt und Fluid in Form von Nebel, Dunst oder feinem Sprühnebel aus der Zerstäubungseinheit (105) ausgestoßen wird und in den Aufnahmeraum gelangt.

2. Vorrichtung nach Anspruch 1, wobei der Bewegungsmechanismus mindestens eine Stange oder einen Kolben umfasst, die mindestens eine Zerstäubungseinheit und das mindestens eine Dichtungselement an der mindestens einen Stange oder dem mindestens einen Kolben angebracht sind und es sich bei der Bewegung von der ersten in die zweite Position um eine axiale Bewegung der Stange oder des Kolbens in Richtung der Öffnung in dem Gehäuse handelt.

3. Vorrichtung nach einem der vorstehenden Ansprüche, wobei das Dichtungselement eine Dichtungsplatte umfasst, die so angepasst ist, dass sie an dem Gehäuse ansetzt, wenn sich der Bewegungsmechanismus in der ersten Position befindet, wodurch das Gehäuse und der Aufnahmeraum abgedichtet werden und verhindert wird, dass Flüssigkeit oder biologisches Material aus dem Aufnahmeraum in das Gehäuse eindringt.

4. Vorrichtung nach dem vorstehenden Anspruch, wobei die Dichtungsplatte an der Stange oder dem Kolben angebracht ist.

5. Vorrichtung nach einem der vorstehenden Ansprüche 3 - 4, wobei die mindestens eine Zerstäubungseinheit an der Dichtungsplatte auf einer zum Gehäuse hin inneren Seite derselben angebracht ist, so dass, wenn sich der Bewegungsmechanismus in der ersten Position befindet, die Zerstäubungseinheit innerhalb des Gehäuses positioniert ist und die Dichtungsplatte in einer Dichtungsposition ist, die verhindert, dass Flüssigkeit in das Gehäuse eindringt.

6. Vorrichtung nach einem der vorstehenden Ansprüche, die zusätzlich eine Rohrleitungseinrichtung zum Zuführen von Flüssigkeit und/oder Gas in die Zerstäubungseinheit umfasst.

7. Vorrichtung nach einem der vorstehenden Ansprüche, wobei die Zerstäubungseinheit eine Zerstäubungsdüse oder -scheibe umfasst.

8. Vorrichtung nach einem der vorstehenden Ansprüche 2 - 7, die ferner mindestens eine Umlenkplatte umfasst, die mit dem distalen Ende des Stabes oder Kolbens verbunden ist, wobei die Umlenkplatte in Richtung einer Strömungsrichtung innerhalb des Aufnahmeraums geneigt ist, so dass ein vorderes Ende der Umlenkplatte näher an der Dichtungsplatte liegt als ein gegenüberliegendes Ende (hinteres Ende) der Umlenkplatte, und wobei die Umlenkplatte so konfiguriert ist, dass sie, wenn die Vorrichtung mit einem Aufnahmeraum verbunden ist, innerhalb der Aufnahmeöffnung an dem Aufnahmeraum positioniert ist.

9. Vorrichtung nach einem der drei vorstehenden Ansprüche, wobei die Umlenkplatte so konfiguriert ist, dass sie in einem Winkel zu der Dichtungsplatte steht.

10. Vorrichtung nach einem der Ansprüche 2 - 9, wobei das Dichtungselement eine erste und eine zweite Dichtungsplatte umfasst, die so angebracht sind, dass die Zerstäubungseinheit zwischen der ersten und der zweiten Dichtungsplatte positioniert ist, und wobei die erste und die zweite Dichtungsplatte das Gehäuse verschließen, wenn sich der Bewegungsmechanismus in der ersten bzw. zweiten Position befindet.

11. Eine Vorrichtung, die Folgendes umfasst:
Ein Behälter zum Transport und/oder zur Lagerung von Lebensmitteln mit Innenflächen, die desinfiziert werden müssen; und
mindestens eine Desinfektionsvorrichtung gemäß jedem der Ansprüche 1 - 10.

12. Vorrichtung oder System nach einem der vorstehenden Ansprüche, die zusätzlich mindestens eine Steuereinheit zur Steuerung der Fluid- und/oder Gaszufuhr in die mindestens eine Zerstäubungseinheit umfasst.

13. Verfahren für das Reinigen und/oder Desinfizieren von Innenflächen eines Raums innerhalb einer Struktur, wie beispielsweise einer Struktur für den Transport und/oder die Lagerung von Lebensmitteln mit folgenden Schritten:
Bereitstellung einer Desinfektionsvorrichtung, die in einem Gehäuse enthalten ist;
Befestigen der Desinfektionsvorrichtung an der Struktur, sodass sie eine Öffnung in der Struktur umschließt und/oder abdeckt, wodurch das abgegebene Fluid in den Raum gelangen kann; und
Zuführen einer Desinfektionsflüssigkeit aus der Desinfektionsvorrichtung in den Raum;
wobei die Desinfektionsvorrichtung eine Zerstäubungseinheit zum Erzeugen und Abgeben von zerstäubtem Fluid in Form von Nebel, Dunst oder feinem Sprühnebel und einen Bewegungsmechanismus zum Bewegen der Zerstäubungseinheit von einer ersten in eine zweite Position umfasst, wobei die Zerstäubungseinheit entsprechend der ersten Position außerhalb des Raums und innerhalb des Gehäuses positioniert ist, so dass die Flüssigkeit dank eines Dichtungselements des Bewegungsmechanismus daran gehindert wird, aus dem Raum in das Gehäuse einzudringen, und entsprechend einer zweiten Position, in der die Zerstäubungseinheit zumindest teilweise innerhalb des Raums positioniert ist, wodurch das von der Zerstäubungseinheit abgegebene Sprüh- oder zerstäubte Fluid in den Raum eintreten kann, um die Innenflächen zu desinfizieren.

14. Verfahren nach dem vorstehenden Anspruch, wobei die Desinfektionsvorrichtung einer Vorrichtung entspricht, wie in einem der Ansprüche 2 -10 festgelegt.

## Revendications

1. Appareil de délivrance d'un fluide d'assainissement dans un espace de réception, l'appareil comprenant :
un logement (108) présentant une ouverture pour un raccordement à une ouverture dans un espace de réception où l'espace de réception est sélectionné parmi un système de stockage d'aliment ou de transport d'aliment, un conduit de ventilation, un arbre, une conduite ou un récipient ;
au moins une unité d'atomisation (105) disposée dans le logement, destinée à générer et délivrer un fin aérosol, un brouillard ou une brume dans l'espace de réception, la au moins une unité d'atomisation (105) étant reliée à un mécanisme mobile ;
un mécanisme mobile (112), dans le logement, présentant une première position et une seconde position, où lorsque le mécanisme se trouve dans la première position, la au moins une unité d'atomisation est positionnée dans le logement et lorsque le mécanisme mobile (112) se trouve dans la seconde position, la au moins une unité d'atomisation (105) est au moins en partie positionnée à l'extérieur du logement, et où un déplacement de la première à la seconde position comprend un déplacement de la au moins une unité d'atomisation (105) vers l'ouverture sur le logement ;
au moins un élément d'étanchéité (103) relié au mécanisme mobile (112) et conçu pour fermer de manière étanche le logement lorsque le logement est relié à l'espace de réception et que le mécanisme mobile (112) se trouve dans la première position ;
où la au moins une unité d'atomisation (105) et le au moins un élément d'étanchéité (103) sont supportés par ledit mécanisme mobile ; et
où l'appareil lorsqu'il est relié à l'espace de réception est actionnable de sorte que la au moins une unité d'atomisation (105) peut se déplacer d'une première position, à laquelle la au moins une unité d'atomisation (105) est positionnée dans le logement, où le au moins un élément d'étanchéité (103) est configuré pour empêcher que du fluide provenant de l'espace de réception n'entre dans ledit logement, à une seconde position, à laquelle l'unité d'atomisation (105) est au moins en partie positionnée à l'extérieur du logement, entrant ainsi dans ledit espace de réception et permettant au fluide sous la forme de brouillard, de brume ou de fin aérosol qui est éjecté depuis ladite unité d'atomisation (105) d'entrer dans ledit espace de réception.

2. Appareil selon la revendication 1, dans lequel le mécanisme mobile comprend au moins une tige ou un piston, où la au moins une unité d'atomisation et ledit au moins un élément d'étanchéité sont montés sur ladite au moins une tige ou ledit au moins un piston, et où un déplacement de la première à la seconde position comprend un déplacement axial de la tige ou du piston vers l'ouverture dans ledit logement.

3. Appareil selon l'une quelconque des revendications précédentes, où le au moins un élément d'étanchéité comprend une plaque d'étanchéité qui est conçue pour s'engager avec le logement lorsque le mécanisme mobile se trouve dans la première position, fournissant une étanchéité entre le logement et l'espace de réception, empêchant que du liquide ou un matériau biologique n'entre dans ledit logement depuis ledit espace de réception.

4. Appareil selon la revendication précédente, où la au moins une plaque d'étanchéité est montée sur ladite au moins une tige ou ledit au moins un piston.

5. Appareil selon l'une quelconque des revendications précédentes 3 et 4, où la au moins une unité d'atomisation est montée sur ladite plaque d'étanchéité sur un côté de celle-ci orienté vers l'intérieur par rapport au logement, de sorte que, lorsque le mécanisme mobile se trouve dans la première position, l'unité d'atomisation est positionnée dans le logement et ladite plaque d'étanchéité se trouve dans une position d'étanchéité qui empêche que du liquide n'entre dans ledit logement.

6. Appareil selon l'une quelconque des revendications précédentes, comprenant en outre un moyen de canalisation, destiné à délivrer du liquide et/ou du gaz dans ladite unité d'atomisation.

7. Appareil selon l'une quelconque des revendications précédentes, dans lequel l'unité d'atomisation comprend une buse ou un disque d'atomisation.

8. Appareil selon l'une quelconque des revendications précédentes 2 à 7, comprenant en outre au moins une plaque de déviation qui est reliée à l'extrémité distale de ladite tige ou dudit piston, où la plaque de déviation est inclinée suivant un sens d'écoulement dans l'espace de réception de sorte qu'une extrémité avant de la plaque de déviation est plus proche de la plaque d'étanchéité qu'une extrémité opposée (extrémité arrière) de la plaque de déviation, et où la plaque de déviation est configurée de sorte que lorsque l'appareil est relié à un espace de réception, la plaque de déviation est positionnée dans l'ouverture de réception sur ledit espace de réception.

9. Appareil selon l'une quelconque des trois revendications précédentes, dans lequel la plaque de déviation est configurée pour être à un certain angle par rapport à ladite plaque d'étanchéité.

10. Appareil selon l'une quelconque des revendications 2 à 9, dans lequel l'élément d'étanchéité comprend une première et une seconde plaques d'étanchéité, où les première et seconde plaques d'étanchéité sont montées sur la tige de sorte que l'unité d'atomisation est positionnée entre les première et seconde plaques d'étanchéité, et où les première et seconde plaques d'étanchéité ferment le logement lorsque le mécanisme mobile se trouve dans la première position et dans la seconde position, respectivement.

11. Système comprenant
- un récipient de stockage et/ou de transport d'aliment présentant des surfaces internes devant être assainies ; et
- au moins un appareil d'assainissement selon l'une quelconque des revendications 1 à 10.

12. Appareil ou système selon l'une quelconque des revendications précédentes, comprenant en outre au moins une unité de commande, destinée à commander une délivrance de fluide et/ou de gaz jusque dans ladite au moins une unité d'atomisation.

13. Procédé de nettoyage et/ou d'assainissement des surfaces internes d'un espace dans une structure, telle qu'une structure de récipient de stockage et/ou de transport d'aliment, le procédé comprenant les étapes consistant à :
fournir un appareil d'assainissement contenu dans un logement ;
attacher l'appareil d'assainissement à la structure, de sorte que l'appareil d'assainissement encercle et/ou couvre une ouverture dans la structure, permettant ainsi au fluide délivré par l'appareil d'assainissement d'entrer dans l'espace ; et
délivrer un fluide d'assainissement depuis l'appareil d'assainissement dans ledit espace ; où l'appareil d'assainissement comprend une unité d'atomisation, destinée à générer et délivrer du fluide atomisé sous la forme de brume, de brouillard ou de fin aérosol, et un mécanisme mobile, destiné à déplacer l'unité d'atomisation d'une première à une seconde position, où dans une première position, l'unité d'atomisation est positionnée à l'extérieur dudit espace et dans ledit logement de sorte que du fluide est empêché d'entrer dans le logement depuis ledit espace par un élément d'étanchéité dudit mécanisme mobile qui empêche que du fluide provenant dudit espace n'entre dans ledit logement, et où dans une seconde position, l'unité d'atomisation est positionnée au moins en partie dans ledit espace, permettant ainsi au fluide atomisé ou pulvérisé délivré par l'unité d'atomisation d'entrer dans ledit espace, en assainissant ainsi ses surfaces internes.

14. Procédé selon la revendication précédente, dans lequel l'appareil d'assainissement est un appareil selon l'une quelconque des revendications 2 à 10.
